# EUROPÄISCHE PATENTSCHRIFT

(11) **EP 1 771 119 B1**
(45) Veröffentlichungstag und Bekanntmachung des Hinweises auf die Patenterteilung: **31.12.2008**
(21) Anmeldenummer: 04738109.0
(22) Anmeldetag: 23.07.2004
(51) Int. Cl.: A61B 17/86, F16B 35/04

(54) **SCHRAUBE**
SCREW
VIS

(43) Veröffentlichungstag der Anmeldung: 11.04.2007
(73) Patentinhaber: Synthes GmbH, 4436 Oberdorf (CH)
(72) Erfinder: SCHLIENGER, André, CH-4144 Arlesheim (CH); SENN, Peter, CH-4437 Waldenburg (CH); BUETTLER, Markus, CH-4702 Oensingen (CH)
(74) Vertreter: Lusuardi, Werther
(86) Internationale Anmeldenummer: PCT/CH2004/000469
(87) Internationale Veröffentlichungsnummer: WO 2006/007739

(56) Entgegenhaltungen:
- WO-A-00/63566
- WO-A-20/04084745
- FR-A- 2 704 140
- US-A- 3 391 720
- US-B1- 6 565 573

## Beschreibung

Die Erfindung bezieht sich auf eine Schraube gemäss dem Oberbegriff des Patentanspruchs 1, insbesondere auf eine Verriegelungsschraube für einen Marknagel mit Querbohrungen.

Die Verriegelung von Marknägeln gehört zum Stand der Technik. Die Einführung von Verriegelungsschrauben in die Querbohrungen eines Marknagels erfolgt entweder mit Hilfe eines bildgebenden Verfahrens (Röntgenkontrolle) oder einer mehr oder weniger komplizierten Zielvorrichtung. In beiden Fällen ist eine gewisse Zielungenauigkeit nicht zu vermeiden, d.h. die Schraubenspitze lässt sich nicht exakt koaxial zur Mittelachse der Querbohrung ausrichten, sondern weicht davon um einen gewissen Betrag ab. Damit die Verriegelungsschraube trotz dieses Zielfehlers in die Querbohrung mündet und durch diese hindurchgebracht werden kann, wird der Aussendurchmesser der Schraube relativ zum Durchmesser der Querbohrung unterdimensioniert. Bleibt die Zielungenauigkeit im Rahmen dieser Unterdimensionierung, so kann die Verriegelungsschraube trotz des Zielfehlers problemlos durch die Querbohrung geführt werden. Allerdings weist nun die Verriegelungsschraube - wegen der Unterdimensionierung - relativ zur Querbohrung ein gewisses Spiel auf.

Dieses Spiel definiert, um welchen Betrag sich die Knochenhauptfragmente, welche mittels Verriegelungsschrauben im entsprechenden Verriegelungsloch fixiert werden, relativ zum Nagel und somit aufgrund der Starrheit des Nagels auch relativ zu anderen mit demselben Nagel befestigten Knochenhauptfragmenten bewegen können. Zusammen mit der Flexibilität des Materials und der Gesamtvorrichtung kann dies kumuliert eine Grösse annehmen, welche eine erfolgreiche Heilung verhindert, oder massgeblich verzögert. Um die Anwendbarkeit der Verriegelung für den Chirurgen zu garantieren, ist dieses Spiel zwar unumgänglich, doch ist es klinisch bei gewissen Indikationen (z.B. im Falle von metaphysären Fragmenten) unerwünscht.

Selbst Nägel mit vollem Querschnitt, welche im Verriegelungsloch ein Innengewinde aufweisen können, sind nicht spielfrei. Das Innengewinde verhindert lediglich, dass der Nagel sich axial auf der Verriegelungsschraube bewegen kann.

Aus der FR 2 704 140 HUBLIN ist eine Knochenschraube gemäss dem Oberbegriff des Anspruchs 1 bekannt.

Ferner ist aus der US 3,391,720 MORSE eine Schraube mit spiralförmig verlaufender Zentralachse bekannt. Die US 6,565,573 FERRANTE zeigt eine weitere Knochenschraube. Hier will die Erfindung Abhilfe schaffen. Der Erfindung liegt die Aufgabe zugrunde, eine Verriegelungsschraube zu schaffen, mit der das vorhandene Spiel zwischen ihr und der Querbohrung in einem Verriegelungs-Marknagel eliminiert werden kann und eine kraftschlüssige Verspannung zwischen der Schraube und dem Marknagel erzeugt werden kann.

Die Erfindung löst die gestellte Aufgabe mit einer Verriegelungsschraube, welche die Merkmale des Anspruchs 1 aufweist.

Der Begriff der Zentrallinie ist definiert durch die Verbindungslinie der Schwerpunkte der axial aufeinanderfolgenden, orthogonalen Querschnittsflächen des Schraubenkerns ohne Berücksichtigung allfälliger Hohlräume und ohne Berücksichtigung des Aussengewindes.

Die durch die Erfindung erreichten Vorteile sind im wesentlichen darin zu sehen, dass dank der erfindungsgemässen Verriegelungsschraube das Spiel zwischen der Querbohrung des Marknagels und Verriegelungsschraube eliminiert werden kann.

Weitere Vorteile sind folgende:
- die Einbringgenauigkeit und der Zeitaufwand für den Chirurgen bleiben im bisherigen Rahmen;
- die Festigkeit der Verriegelungsschraube bleibt erhalten;
- die Extraktion bei einem allfälligen Schraubenbruch ist gewährleistet;
- die durch die Hohlräume bedingte materialmässige Schwächung des Schraubenkernes erleichtert dessen Vortrieb durch eine verengte Stelle, z.B. in eine Querbohrung eines Marknagels, weil die keine geradlinige Zentrallinie aufweisende Schraube sich bis zu einem gewissen Grad radial deformieren kann; und
- aufgrund der axialen Kannulierung auch ein Führungsdraht verwendet werden kann.

Die Zentrallinie der Schraube kann helix- oder schraubenlinienförmig verlaufen. Dadurch ergibt sich eine axiale Auslenkung der Schraube, welche eine graduelle Aufhebung des Spiels bis zu einem allmählichen Aufbau der Verspannung führt.

Die Zentrallinie kann in einer einzigen Ebene liegen, wodurch sich eine besonders einfache Herstellung der Schraube ergibt.

Vorzugsweise ist am kopfseitigen Ende der Schraube eine Markierung angebracht. Die Markierung zeigt vorteilhafterweise die Orientierung der Ebene, in welcher allfällige Wendepunkte der Zentrallinie liegen, an.

Die Zentrallinie kann auch nicht in einer Ebene liegen. Das Hindurchwinden der Schraube wird dadurch einfacher und die Verspannungspunkte liegen nicht auf einer Linie.

Die Zentrallinie kann beispielsweise aus mehreren gegeneinander verschobenen Geraden gebildet sein.

Bei einer besonderen Ausführungsform wird die Mantelfläche des Schraubenkerns nicht aus einer Schar von parallelen Geraden gebildet. Die Mantelfläche entspricht somit nicht der Mantelfläche eines geraden Kreiszylinders, sondern einem gemäss der nichtgeradlinigen Zentrallinie deformierten Kreiszylinder.

Das Volumen Vₕₒₕₗ des ein oder mehrere Hohlräume aufweisenden Schraubenkerns beträgt vorteilhafterweise weniger als 95 % des Volumens Vᵥₒₗₗ des von der Mantelfläche des Schraubenkerns umschlossenen Körpers. Typischerweise ist Vₕₒₕₗ < 0,90 Vᵥₒₗₗ und vorzugsweise Vₕₒₕₗ < 0,80 Vᵥₒₗₗ.

Die Hohlräume im Schraubenkern - unter Ausschluss eines allfälligen axialen Austritts eines Hohlraumes an der Spitze der Schraube - sollte vorteilhafterweise höchstens 1 % und vorzugsweise 0 % der Mantelfläche durchdringen.

Die Spitze der Schraube kann sich verjüngen und das kopfseitige Ende kann Mittel zum Antrieb der Schraube aufweisen.

Bei einer besonderen Ausführungsform besitzt der Schraubenkern keine Rotations-Symmetrieachse.

Bei einer weiteren Ausführungsform weist eine zwischen dem Zentrum des kopfseitigen Endes und dem Zentrum der Spitze verlaufende Gerade mindestens stellenweise einen Abstand x > 0 von der Zentrallinie auf. Der Abstand "x" gehorcht vorteilhafterweise der Bedingung 0,02 d < x < 0,6 d und vorzugsweise der Bedingung 0,1 d < x < 0,4 d, wobei "d" dem Durchmesser des Aussengewindes entspricht. Der Abstand "x" sollte vorteilhafterweise - mindestens auf einer axialen Teilstrecke - grösser als 0,05 mm, vorzugsweise grösser als 0,50 mm sein.

Die zwischen dem kopfseitigen Ende und der Spitze verlaufende Zentrallinie weist die Länge L auf und ist in drei Abschnitte A, B und C unterteilt, wobei sich
A) Abschnitt A vom kopfseitigen Ende (5) um den Betrag 0,10 L bis 0,25 L gegen die Spitze hin erstreckt;
B) Abschnitt B von der Spitze um den Betrag 0,10 bis 0,25 L gegen das kopfseitige Ende hin erstreckt; wobei
C) Abschnitt C zwischen den beiden Abschnitten A und B angeordnet ist und die Länge C = (L - A - B) aufweist; und
D) die Zentrallinie in den Abschnitten A und B geradlinig und koaxial zueinander verläuft.

Dadurch ergibt sich der Vorteil, dass die Verriegelung in der gegenüberliegenden Kortikalis durch Rotationsbewegung um die Zentrallinie und die Verriegelung in der kopfseitigen Kortikalis möglichst bohrachsengerecht erfolgt.

Bei einer weiteren Ausführungsform ist die Zentrallinie im Abschnitt C S-förmig gekrümmt oder exzentrisch. Sie weist zudem vorzugsweise im Abschnitt C einen Wendepunkt auf. Bei einer weiteren Ausführungsform weist die Zentrallinie vorzugsweise im Abschnitt C mindestens zwei Wendepunkte im Abstand y voneinander auf.

Bei einer besonderen Ausführungsform ist die Wandstärke W des Schraubenkerns im Bereich der Kannulierung grösser als 0,05 d, vorzugsweise grösser als 0,1 d. Vorteilhafterweise ist die Wandstärke W des Schraubenkerns im Bereich der Kannulierung kleiner als 0,8 d, vorzugsweise kleiner als 0,6 d.

Die erfindungsgemässe Schraube kann zusammen mit einem Verriegelungsmarknagel verwendet werden, welcher mindestens eine Querbohrung aufweist, welche ein Querschnittsprofil P mit einer maximalen Ausdehnung a in Richtung der Zentrallinie gemessen und einer maximalen Ausdehnung b senkrecht zu a gemessen aufweist, wobei einerseits a > b und anderseits a > d < b gilt. Das Querschnittsprofil P kann aber auch kreisförmig mit a = b sein. Vorteilhafterweise wird die Bedingung 0,70 b < d < 0,95 b und vorzugsweise 0,8 b < d < 0,9 b erfüllt.

Der Abstand x gehorcht vorteilhafterweise der Bedingung x < (b - d + 1 mm), wobei "b" der Durchmesser der Querbohrung in mm und "d" der Durchmesser des Schraubenkernes in mm ist.

Der Abstand x sollte vorteilhafterweise der Bedingung 0,05 (b - d) < x < 0,35 (b - d) und vorzugsweise der Bedingung 1,5 (b - d) < x < 2,2 (b - d) gehorchen, wobei "d" der Durchmesser des Aussengewindes in mm ist.

Der Durchmesser D sollte der Bedingung 0,02 d < D < 0,60 d, vorzugsweise der Bedingung 0,1 d < x < 0,4 d gehorchen, wobei "d" dem Durchmesser des Aussengewinde entspricht.

Der.Abstand y zwischen zwei benachbarten Wendepunkten sollte im wesentlichen der Bedingung D = n y gehorchen, wobei n eine ungerade Zahl ist und D der Durchmesser des Marknagels ist.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Ansicht einer erfindungsgemässen Schraube;
Fig. 2 einen Längsschnitt durch die Schraube nach Fig. 1; und
Fig. 3 einen Längsschnitt durch einen Verriegelungsmarknagel mit einer Querbohrung, in welcher die Schraube nach Fig. 1 eingeführt ist.

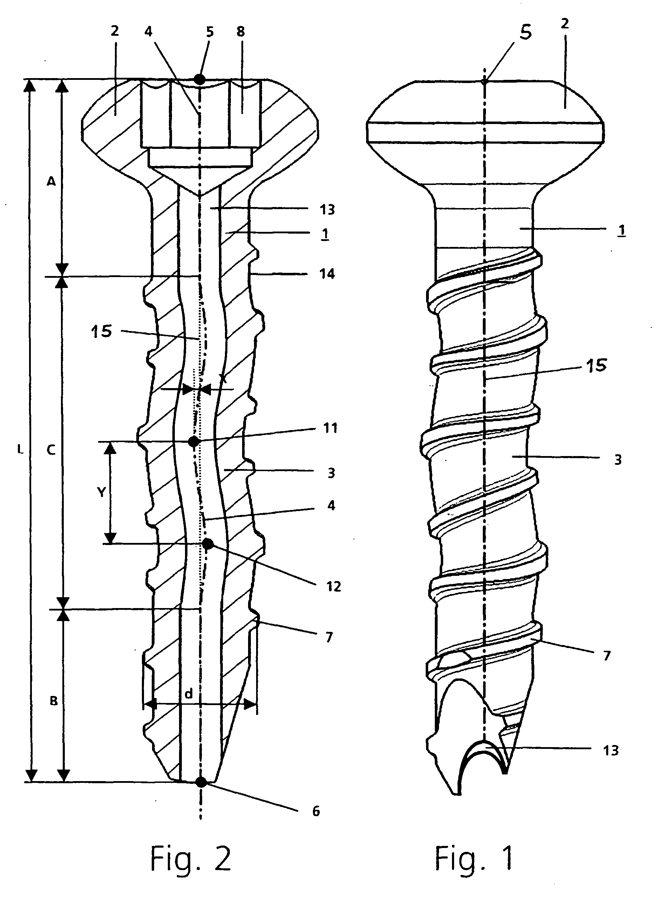

Die in den Fig. 1 und 2 gezeigte Schraube umfasst einen Schraubenkopf 2 mit einem Innensechskant 8, einem Schraubenkern 3 mit einem Aussengewinde 7 und einer Zentrallinie 4, welche sich vom kopfseitigen Ende 5 zur Spitze 6 der Schraube erstreckt. Die Zentrallinie 4 ist keine Gerade wie bei den üblichen geraden Schrauben, sondern besteht im gezeigten Ausführungsbeispiel (im weiter unten definierten Längenbereich C) aus einer Schlangenlinie, die in der Zeichenebene liegt und zwei Wendepunkte 11,12 besitzt. Die durch das Zentrum des kopfseitigen Endes 5 und der Spitze 6 verlaufende Gerade 15 weicht deshalb stellenweise von der Zentrallinie 4 um den variablen Betrag x ab. Im gezeigten Ausführungsbeispiel beträgt die maximale Abweichung von x (in den Wendepunkten der Zentrallinie 4 gemessen) 0,2 mm.

Zwischen dem Zentrum des kopfseitigen Endes 5 und der Spitze 6, welche den Abstand L zueinander aufweisen, ist die Zentrallinie 4 in drei Abschnitte A, B und C unterteilt. Abschnitt A erstreckt sich vom Zentrum 5 am Schraubenkopf 2 um den Betrag 1/6 L gegen die Spitze 6 am freien Ende des Schraubenkernes 3 hin und verläuft geradlinig. Abschnitt B erstreckt sich von der Spitze 6 am freien Ende des Schraubenkernes 3 um den Betrag 1/6 L gegen das Zentrum des kopfseitigen Endes 5 am Schraubenkopf 2 hin und verläuft ebenfalls geradlinig sowie koaxial zum Abschnitt A. Abschnitt C ist zwischen den beiden Abschnitten A und B angeordnet und weist wie oben beschrieben eine Krümmung auf.

Der Hohlraum 13 in Form einer axial verlaufenden Kannulierung kann statt schlangenlinien- oder helixförmig auch geradlinig ausgebildet sein, was einfacher in der Herstellung ist.

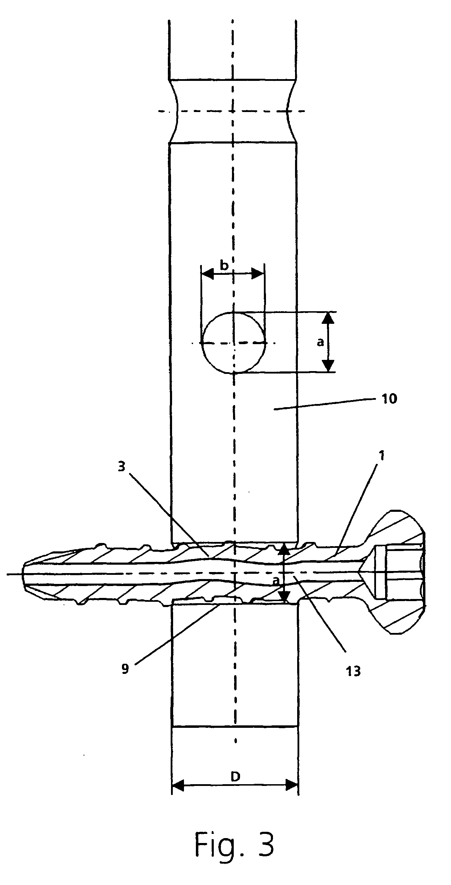

In Fig. 3 ist gezeigt wie die Schraube 1 in der Querbohrung 9 eines Marknagels 10 eingeführt ist. Das Aussengewinde 7 des Schraubenkerns 3 weist dabei einen Durchmesser "d" (Fig. 2) auf, der kleiner ist als das Mass "a" der Querbohrung 9.

Im Folgenden wird noch kurz die Technik des Eindrehens der Verriegelungsschraube in die Querbohrung eines Marknagels erläutert:
a) der Chirurg dreht die Schraube 1 standardmässig durch die Querbohrung 9 des Marknagels 10 ein;
b) die relativ dünne und weiche Kortikalis gibt beim Hindurchwinden der Schraube 1 nach, so dass über die Kortikalisdicke keine Verspannung erfolgt;
c) im Bereich des Marknagels 10 streckt und verspannt sich die Schraube 1 etwas, wegen der Reaktion der Querbohrungswandung, so dass eine erhöhte Eindrehkraft sowie eine erhöhte Haltekraft resultiert;
d) Die Schraube 1 windet sich durch die Eintrittsöffnung der Querbohrung 9 des Marknagels 10. Da der Durchmesser D des Marknagels 10 grösser ist als der Abstand y zwischen den beiden Wendepunkten 11,12 verspannt sich die Schraube 1 in der Querbohrung 9. Die Schraube 1 wird durch das Eindrehmoment, resp. durch die Vorschubkraft des Chirurgen spätestens beim Fassen in der Gegenkortikalis in eine elastische Deformation gezwungen, was zu einer zusätzlichen Verspannung der Schraube 1 und zur winkelstabilen Verriegelung des Marknagels 10 führt.

## Patentansprüche

1. Schraube (1) mit einem kopfseitigen Ende (5), einer Spitze (6), einem Schraubenkern (3) mit einem oder mehreren Hohlräumen (13) und mit einer Mantelfläche (14), welche ein Aussengewinde (7) trägt, sowie einer Zentrallinie (4) gebildet durch die Verbindungslinie der Schwerpunkte der axial aufeinanderfolgenden, orthogonalen Querschnittsflächen des Schraubenkerns (3) ohne Berücksichtigung allfälliger Hohlräume (13) und ohne Berücksichtigung des Aussengewindes (7), wobei
die zwischen dem kopfseitigen Ende (5) und der Spitze (6) verlaufende Zentrallinie (4) die Länge L aufweist und in drei Abschnitte A, B und C unterteilt ist, wobei sich
A) Abschnitt A vom kopfseitigen Ende (5) um den Betrag 0,10 L bis 0,25 L gegen die Spitze (6) hin erstreckt;
B) Abschnitt B von der Spitze (6) um den Betrag 0,10 bis 0,25 L gegen das kopfseitige Ende (5) hin erstreckt; wobei
C) Abschnitt C zwischen den beiden Abschnitten A und B angeordnet ist und die Länge C = (L - A - B) aufweist; und
D) die Zentrallinie (4) in den Abschnitten A und B geradlinig und koaxial zueinander verläuft, wobei
E) der Schraubenkern (3) einen Hohlraum (13) in Form einer axialen Kannulierung aufweist, welche die Schraube (1) durchgehend durchbohrt, **dadurch gekennzeichnet, dass** die Zentrallinie im Abschnitt C nicht geradlinig ist*.*

2. Schraube (1) nach Anspruch 1, **dadurch gekennzeichnet, dass** die Zentrallinie (4) helix- oder schraubenlinienförmig verläuft.

3. Schraube nach Anspruch 1, **dadurch gekennzeichnet, dass** die Zentrallinie (4) in einer Ebene liegt.

4. Schraube (1) nach einem der Ansprüche 1 bis 3, **dadurch gekennzeichnet, dass** am kopfseitigen Ende (5) der Schraube (1) eine Markierung angebracht ist.

5. Schraube (1) nach den Ansprüchen 3 und 4, **dadurch gekennzeichnet, dass** die Markierung die Orientierung der Ebene, in welcher allfällige Wendepunkte der Zentrallinie (4) liegen, markiert.

6. Schraube (1) nach einem der Ansprüche 1 oder 2, **dadurch gekennzeichnet, dass** die Zentrallinie (4) nicht in einer Ebene liegt.

7. Schraube (1) nach Anspruch 1, **dadurch gekennzeichnet, dass** die Zentrallinie (4) aus mehreren gegeneinander verschobenen Geraden gebildet ist.

8. Schraube (1) nach einem der Ansprüche 1 bis 7, **dadurch gekennzeichnet, dass** die Mantelfläche (14) nicht aus einer Schar von parallelen Geraden gebildet ist.

9. Schraube (1) nach einem der Ansprüche 1 bis 8, **dadurch gekennzeichnet, dass** das Volumen Vₕₒₕₗ des ein oder mehrere Hohlräume (13) aufweisenden Schraubenkerns (3) weniger als 95 % des Volumens Vᵥₒₗₗ des von der Mantelfläche (14) des Schraubenkerns (3) umschlossenen Körpers beträgt.

10. Schraube (1) nach Anspruch 9, **dadurch gekennzeichnet, dass** Vₕₒₕₗ < 0,90 Vᵥₒₗₗ und vorzugsweise dass Vₕₒₕₗ < 0,80 Vᵥₒₗₗ ist.

11. Schraube (1) nach einem der Ansprüche 1 bis 10, **dadurch gekennzeichnet, dass** die Hohlräume (13) - unter Ausschluss eines allfälligen axialen Austritts eines Hohlraumes (13) an der Spitze (6) - höchstens 1 % und vorzugsweise 0 % der Mantelfläche (14) durchdringen.

12. Schraube (1) nach einem der Ansprüche 1 bis 11, **dadurch gekennzeichnet, dass** ihre Spitze (6) verjüngt ist.

13. Schraube (1) nach einem der Ansprüche 1 bis 12, **dadurch gekennzeichnet, dass** das kopfseitige Ende (5) Mittel (8) zum Antrieb der Schraube (1) aufweist.

14. Schraube (1) nach einem der Ansprüche 1 bis 13, **dadurch gekennzeichnet, dass** der Schraubenkern (3) keine Rotations-Symmetrieachse besitzt.

15. Schraube (1) nach einem der Ansprüche 1 bis 14, **dadurch gekennzeichnet, dass** eine zwischen dem Zentrum des kopfseitigen Endes (5) und dem Zentrum der Spitze (6) verlaufende Gerade (15) mindestens stellenweise einen Abstand x > 0 von der Zentrallinie (4) aufweist.

16. Schraube (1) nach Anspruch 15, **dadurch gekennzeichnet, dass** das Aussengewinde (7) den Durchmesser "d" aufweist und der Abstand "x" der Bedingung 0,02 d < x < 0,6 d, vorzugsweise 0,1 d < x < 0,4 d gehorcht.

17. Schraube (1) nach Anspruch 15 oder 16, **dadurch gekennzeichnet, dass** der Abstand "x" mindestens auf einer axialen Teilstrecke grösser als 0,05 mm, vorzugsweise grösser als 0,50 mm ist.

18. Schraube (1) nach einem der Ansprüche 1 bis 17, **dadurch gekennzeichnet, dass** die Zentrallinie (4) im Abschnitt C S-förmig gekrümmt oder exzentrisch ist.

19. Schraube (1) nach einem der Ansprüche 1 bis 18, **dadurch gekennzeichnet, dass** die Zentrallinie (4) im Abschnitt C einen Wendepunkt (11) aufweist.

20. Schraube (1) nach einem der Ansprüche 1 bis 19, **dadurch gekennzeichnet, dass** die Zentrallinie (4) im Abschnitt C mindestens zwei Wendepunkte (11;12) im Abstand y voneinander aufweist.

21. Schraube (1) nach einem der Ansprüche 1 bis 20, **dadurch gekennzeichnet, dass** das Aussengewinde (7) den Durchmesser "d" aufweist und die Wandstärke W des Schraubenkerns (3) im Bereich der Kannulierung grösser als 0,05 d, vorzugsweise grösser als 0,1 d ist.

22. Schraube (1) nach einem der Ansprüche 1 bis 22, **dadurch gekennzeichnet, dass** das Aussengewinde (7) den Durchmesser "d" aufweist und die Wandstärke W des Schraubenkerns (3) im Bereich der Kannulierung kleiner als 0,8 d, vorzugsweise kleiner als 0,6 d ist.

23. Schraube (1) nach einem der Ansprüche 1 bis 22, **dadurch gekennzeichnet, dass** die Schraube (1) einen Schraubenkopf (2) mit einem Innensechskant (8) und einen Schraubenkern (3) mit einem Aussengewinde (7) umfasst.

## Claims

1. Screw (1) with a head end (5), a tip (6), a screw core (3) with one or more cavities (13) and with a peripheral surface (14), which carries an external thread (7), as well as a central line (4), which is formed by the line connecting the centers of gravity of the axially successive, orthogonal cross-sectional areas of the screw core (3), disregarding any cavities (13) and disregarding the external thread (7), whereby
the central line (4), extending between the head end (5) and the tip (6), has the length L and is divided into three sections A, B and C, whereby
A) section A extending from the head end (5) by the amount of 0.10 L to 0.25 L in the direction of the tip (6),
B) section B extending from the tip (6) by the amount of 0.10 to 0.25 L in the direction of the head end (5), whereby
C) section C extending between the two sections A and B with the proviso that the length C = (L - A - B); and
D) the central line (4) in the sections A and B being rectilinear and extending coaxially with one another, whereby
E) the screw core (3) has a cavity (13) in the form of an axial cannulation, which passes through the screw (1) continuously,
**characterized in that** the central line is not rectilinear in the section C.

2. The screw (1) of claim 1, **characterized in that** the central line (4) proceeds helically or spirally.

3. The screw of claim 1, **characterized in** the central line (4) lies in one plane.

4. The screw (1) of one of the claims 1 to 3, **characterized in that** a marking is provided at the head end (5) of the screw (1).

5. The screw (1) of claims 3 and 4, **characterized in that** the marking marks the orientation of the plane, in which any points of inflection of the central line (4) are located.

6. The screw (1) of claim 1 or 2, **characterized in that** the central line (4) does not lie in one plane.

7. The screw (1) of claim 1, **characterized in that** the central line (4) is formed from several straight lines, which are misaligned with one another.

8. The screw (1) of one of the claims 1 to 7, **characterized in that** the circumferential surface (14) is not formed from a family of parallel straight lines.

9. The screw (1) of one of the claims 1 to 8, **characterized in that** the volume Vₕₒₕₗ of the screw core (3), having one or more cavities (13), is less than 95% of the volume Vᵥₒₗₗ of the body enclosed by the circumferential surface (14) of the screw core (3).

10. The screw (1) of claim 9, **characterized in that** Vₕₒₕₗ < 0.90 Vᵥₒₗₗ and preferably that Vₕₒₕₗ < 0.80 Vᵥₒₗₗ.

11. The screw (1) of one of the claims 1 to 10, **characterized in that** the cavities (13), excluding any axial outlet of a cavity (13) at a tip (6), penetrate not more than 1% and preferably 0% of the circumferential surface (14).

12. The screw (1) of one of the claims 1 to 11, **characterized in that** its tip (6) is tapered.

13. The screw (1) of one of the claims 1 to 12, **characterized in that** the head end (5) has means (8) for driving the screw (1).

14. The screw (1) of one of the claims 1 to 13, **characterized in that** the screw core (3) does not have an axis of rotational symmetry.

15. The screw (1) of one of the claims 1 to 14, **characterized in that** a straight line (15), extending between the center of the head end (5) and the center of the tip (6), is at a distance of x > 0 from the center line (4) at least in places.

16. The screw (1) of claim 15, **characterized in that** the external thread (7) has the diameter "d" and the distance "x" fulfills the condition that 0.02 d < x < 0.6 d and preferably that 0.1 d < x < 0.4 d.

17. The screw (1) of claims 15 or 16, **characterized in that** the distance "x" is greater than 0.05 mm and preferably greater than 0.50 mm at least on a partial axial segment.

18. The screw (1) of one of the claims 1 to 17, **characterized in that** the central line (4) is curved S-shaped or is eccentric in section C.

19. The screw (1) of one of the claims 1 to 18, **characterized in that** the central line (4) has a point of inflection (11) in section C.

20. The screw (1) of one of the claims 1 to 19, **characterized in that** the central line (4) has two points of inflection (11; 12) at a distance of y from one another in section C.

21. The screw (1) of one of the claims 1 to 20, **characterized in that** the external thread (7) has a diameter "d" and the thickness W of the wall of the screw core (3) in the region of the cannulation is greater than 0.05 d and preferably greater than 0.1 d.

22. The screw (1) of one of the claims 1 to 21, **characterized in that** the external thread (79 has a diameter "d" and the thickness W of the wall of the screw core (3) in the region of the cannulation is smaller than 0.8 d and preferably smaller than 0.6 d.

23. The screw (1) of one of the claims 1 to 22, **characterized in that** the screw (1) comprises a screw head (2) with a hexagon socket (8) and a screw core (3) with an external thread (7).

## Revendications

1. Vis (1) comportant une extrémité (5) côté tête, une pointe (6), un noyau de vis (3) qui comporte un ou plusieurs espaces creux (13) et une surface d'enveloppe (14) qui porte un filetage extérieur (7), ainsi qu'une ligne centrale (4) formée par la ligne de raccordement des centres de gravité des sections orthogonales du noyau de vis (3), qui se succèdent axialement, sans tenir compte ni d'éventuels espaces creux (3) ni du filetage extérieur (7),
la ligne centrale (4), qui s'étend entre l'extrémité (5) côté tête et la pointe (6), ayant la longueur L et étant subdivisée en trois portions A, B et C,
A) la portion A s'étendant de l'extrémité (5) côté tête vers la pointe (6) sur une étendue allant de 0,10 L à 0,25 L ;
B) la portion B s'étendant de la pointe (6) vers l'extrémité (5) côté tête sur une étendue allant de 0,10 à 0,25 L ;
C) la portion C étant disposée entre les deux portions A et B et ayant la longueur C = (L-A-B) ; et
D) la ligne centrale (4) s'étendant de façon rectiligne dans les portions A et B et coaxialement à celles-ci,
E) le noyau de vis (3) comportant un espace creux (13) ayant la forme d'une cannelure axiale qui traverse la vis (1) de bout en bout,
**caractérisée en ce que** la ligne centrale n'est pas rectiligne dans la portion C.

2. Vis selon la revendication (1), **caractérisée en ce que** la ligne centrale (4) s'étend en forme d'hélice ou de filet de vis.

3. Vis selon la revendication 1, **caractérisée en ce que** la ligne centrale (4) est située dans un plan.

4. Vis (1) selon l'une des revendications 1 à 3, **caractérisée en ce qu'**un repère est marqué à l'extrémité (5) côté tête de la vis (1).

5. Vis (1) selon les revendications 3 et 4, **caractérisée en ce que** le repère indique l'orientation du plan dans lequel se trouvent d'éventuels points d'inflexion de la ligne centrale (4).

6. Vis (1) selon l'une des revendications 1 ou 2, **caractérisée en ce que** la ligne centrale (4) ne se trouve pas dans un plan.

7. Vis (1) selon la revendication 1, **caractérisée en ce que** la ligne centrale (4) est constituée de plusieurs droites décalées l'une par rapport à l'autre.

8. Vis (1) selon l'une des revendications 1 à 7, **caractérisée en ce que** la surface d'enveloppe (14) n'est pas constituée d'un système de droites parallèles.

9. Vis (1) selon l'une des revendications 1 à 8, **caractérisée en ce que** le volume V_{creux} du noyau de vis (3), comportant une ou plusieurs espaces creux (13), est inférieur à 95% du volume Vₚₗₑᵢₙ du corps entouré par la surface d'enveloppe (14) du noyau de vis (3).

10. Vis (1) selon la revendication 9, **caractérisée en ce que** V_{creux} < 0,90 Vₚₗₑᵢₙ et avantageusement **en ce que** V_{creux} < 0,80 Vₚₗₑᵢₙ.

11. Vis (1) selon l'une des revendications 1 à 10, **caractérisée en ce que** les espaces creux (13) - à l'exclusion d'une éventuelle sortie axiale d'un espace creux (13) à la pointe (6) - pénètrent dans 1% maximum, et avantageusement 0%, de la surface d'enveloppe (14).

12. Vis (1) selon l'une des revendications 1 à 11, **caractérisée en ce que** sa pointe (6) est amincie.

13. Vis (1) selon l'une des revendications 1 à 12, **caractérisée en ce que** l'extrémité (5) côté tête comporte des moyens (8) pour l'entraînement de la vis (1).

14. Vis (1) selon l'une des revendications 1 à 13, **caractérisée en ce que** le noyau de vis (3) ne possède aucun axe de symétrie de révolution.

15. Vis (1) selon l'une des revendications 1 à 14, **caractérisée en ce qu'**une droite (15), s'étendant entre le centre de l'extrémité (5) côté tête et le centre de la pointe (6), se trouve au moins par endroits à une distance x > 0 par rapport à la ligne centrale (4).

16. Vis (1) selon la revendication 15, **caractérisée en ce que** le filetage extérieur (7) présente le diamètre 'd' et la distance 'x' satisfait à la condition 0,02 d < x < 0,6 d, avantageusement 0,1 d < x < 0,4 d.

17. Vis (1) selon la revendication 15 ou 16, **caractérisée en ce que** la distance 'x' est supérieure à 0,05 mm, avantageusement supérieure à 0,50 mm, au moins sur une partie de l'étendue axiale.

18. Vis (1) selon l'une des revendications 1 à 17, **caractérisée en ce que** la ligne centrale (4) est incurvée en S ou excentrique dans la portion C.

19. Vis (1) selon l'une des revendications 1 à 18, **caractérisée en ce que** la ligne centrale (4) possède un point d'inflexion (11) dans la portion C.

20. Vis (1) selon l'une des revendications 1 à 19, **caractérisée en ce que** la ligne centrale (4) comporte dans la portion C au moins deux points d'inflexion (11 ; 12) espacés de la distance y.

21. Vis (1) selon l'une des revendications 1 à 20, **caractérisée en ce que** le filetage extérieur (7) présente le diamètre 'd' et l'épaisseur W du noyau de vis (3), au niveau de la cannelure, est supérieure à 0,05 d, avantageusement supérieure à 0,1 d.

22. Vis (1) selon l'une des revendications 1 à 22, **caractérisée en ce que** le filetage extérieur (7) présente le diamètre 'd' et l'épaisseur W du noyau de vis (3), au niveau de la cannelure, est inférieure à 0,8 d, avantageusement inférieure à 0,6 d.

23. Vis (1) selon l'une des revendications 1 à 22, **caractérisée en ce que** la vis (1) comporte une tête de vis (2) à six pans creux (8) et un noyau de vis (3) avec un filetage extérieur (7).
